(19) Europäisches Patentamt
European Patent Office
Office européen des brevets

(11) **EP 4 218 418 A1**

(12) **EUROPEAN PATENT APPLICATION**
published in accordance with Art. 153(4) EPC

(43) Date of publication:
**02.08.2023 Bulletin 2023/31**

(21) Application number: **21872939.0**

(22) Date of filing: **24.09.2021**

(51) International Patent Classification (IPC):
*A23K 20/10* (2016.01)    *A23K 10/30* (2016.01)
*A61K 36/38* (2006.01)    *A61P 33/10* (2006.01)
*A61P 33/04* (2006.01)

(52) Cooperative Patent Classification (CPC):
**A23K 10/30; A23K 20/10; A61K 36/38;
A61P 33/04; A61P 33/10;** Y02P 60/87

(86) International application number:
**PCT/KR2021/013035**

(87) International publication number:
**WO 2022/065911 (31.03.2022 Gazette 2022/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.09.2020 KR 20200125245**

(71) Applicant: **CJ Cheiljedang Corporation
Seoul 04560 (KR)**

(72) Inventors:
• **LEE, Kyung Min
Seoul 04560 (KR)**
• **KIM, Hwi-Jea
Seoul 04560 (KR)**
• **PARK, Min Ah
Seoul 04560 (KR)**
• **SON, Kyuyeol
Seoul 04560 (KR)**

(74) Representative: **Bates, Philip Ian
Reddie & Grose LLP
The White Chapel Building
10 Whitechapel High Street
London E1 8QS (GB)**

Remarks:
The complete document including Reference
Table(s) and the Sequence Listing(s) can be
downloaded from the EPO website

(54) **ANTI-COCCIDIAL COMPOSITION COMPRISING MANGOSTEEN AND USES THEREOF**

(57)    The present application relates to an anti-coccidial composition comprising mangosteen, and the uses thereof. The composition comprising mangosteen according to an example has excellent effect of directly killing protozoa which can induce coccidiosis, effect of inhibiting cell penetration by protozoa and/or effect of inhibiting intracellular protozoan proliferation, and has excellent in vivo coccidiosis prevention, relief and therapeutic effects.

[FIG. 1]

EP 4 218 418 A1

**Description**

[TECHNICAL FIELD]

Cross-reference to related application(s)

The present application claims the benefit of the priority based on Korean Patent Application No. 10-2020-0125245 filed on September 25, 2020, and the entire contents disclosed in the document of the corresponding Korean patent application are incorporated as a part of the present description.

The present application relates to an anticoccidial composition comprising a mangosteen and uses thereof.

[BACKGROUND ART]

Coccidiosis is an intestinal-related disease caused by a protozoan parasite belonging to the phylum apicomplexan called *Eimeria,* and when affected with coccidiosis, symptoms of digestive disorders, diarrhea and weight loss are shown, and furthermore, death of livestock is caused, and therefore, it has a major economic impact on farms around the world (Williams RB. A compartmentalised model for the estimation of the cost of coccidiosis to the world's chicken production industry. Int J Parasitol. 1999; 29(8): 1209-1229).

Over the past few years, many researchers have developed anticoccidial agents such as ionophores or chemically synthetic compounds, which can prevent oocyst cell wall formation or asexual and sexual reproduction of protozoa as therapeutic agents for treating coccidiosis. However, side effects such as appearance of protozoa with drug resistance due to the long-term use of a shuttle program that alternately treats the ionophores and chemically synthetic compounds and the like occurred.

In particular, antibiotics accumulated in animals due to misuse and abuse of antibiotics are a serious problem as humans consume antibiotics through meat, and therefore, antibiotic administration are being banned in many countries around the world due to the problem of antibiotic residues in livestock products. Therefore, there is an urgent need to develop and research alternatives to conventional anticoccidial agents that exhibit side effects such as emergence and internal residue of strains resistant to drugs.

[PRIOR ART]

[PATENT DOCUMENT]

(Patent document 1) U.S. Patent Publication No. 2008-0160000

[DISCLOSURE]

[TECHNICAL PROBLEM]

One embodiment of the present application provides a feed composition for preventing or alleviating coccidiosis, comprising a mangosteen.

Another embodiment of the present application provides a pharmaceutical composition for preventing or alleviating coccidiosis, comprising a mangosteen.

Other embodiment of the present application provides an antiprotozoal composition against an *Eimeria* sp. protozoan, comprising a mangosteen.

Other embodiment of the present application provides a method for preventing, alleviating or treating coccidiosis, comprising a step of administering the composition to an animal.

Other embodiment of the present application provides a use of a mangosteen for the manufacture of a composition (for example, feed composition, pharmaceutical composition) for preventing, alleviating and/or treating coccidiosis or an antiprotozoal composition; a use of a mangosteen for using in preventing, alleviating and/or treating coccidiosis; and/or a use of a mangosteen for using in antiprotozoan against an *Eimeria* sp. protozoan (for example, *Eimeria* sp. protozoa killing; and/or cell invasion and/or propagation inhibition of *Eimeria* sp. protozoa).

[TECHNICAL SOLUTION]

The composition comprising a mangosteen according to one embodiment may have excellent anticoccidial activity and/or antiprotozoal activity against protozoa inducing coccidiosis.

In the present application, the excellent anticoccidial efficacy (activity, effect) may mean at least one (for example, any one, 2 or more kinds, 3 or more kinds, or all) selected from the group consisting of the following (1) to (5):

(1) a higher anticoccidial index (ACI) compared to the control group;
(2) reduced mortality, lesion score (for example, appendix lesion score) and/or fecal oocyst excretion amount compared to the control group when administered to a coccidiosisinduced animal subject;
(3) inhibition of weight loss by induction of coccidiosis;
(4) higher insecticidal activity against protozoa which induce coccidiosis compared to the control group; and
(5) a higher inhibitory effect of cell invasion of protozoa which induce coccidiosis and/or propagation of the protozoa in cells compared to the control group.

In the present application, the control group may mean a negative control group (a group in which nothing is treated or water and/or a buffer treatment group) and/or a positive control group comprising a conventionally known anticoccidial agent (for example, diclazuril, salinomycin and/or gallic acid).

The composition according to one embodiment may have at least one (for example, one or more kinds, 2 or more kinds, 3 or more kinds, 4 or more kinds, 5 or more kinds or all of 6 kinds) characteristics selected from the group consisting of the following (1) to (6), and its characteristic may be more excellent than the control group:

(1) excellent anticoccidial activity;
(2) excellent antiprotozoal effect against protozoa which induce coccidiosis;
(3) excellent acid resistance;
(4) excellent heat resistance;
(5) excellent in vivo stability and/or safety; and
(6) excellent weight gain improvement effect.

The composition according to one embodiment has excellent acid resistance and/or heat resistance, thereby maintaining the excellent anticoccidial activity for a long period of time when administered to a body, and has in vivo stability, thereby maintaining the excellent anticoccidial activity even in an environment in various temperature and/or pH ranges, and it may be applicable for various products (for example, feed additives), and the storage stability may be excellent.

The composition according to one embodiment is not absorbed by other tissue and organs (for example, blood, liver, kidney and/or spleen, etc.) other than intestines when administered in vivo, and therefore, has a low residue in a body, and thus, it may have excellent safety in vivo.

In one embodiment, the excellent weight gain improvement effect may mean an excellent effect of increasing a body weight of a subject when administered to the subject, and in one embodiment, the weight gain may mean daily weight gain, and the subject may be a subject in which coccidiosis is induced.

The composition according to one embodiment exhibits the anticoccidial activity equivalent or more than conventional known anticoccidial agents (for example, sulfa agents such as sulfaquinoxaline, sulfachloropyrazine and sulfamethazine, polyether ionophore antibiotics such as salinomycin and monensin sodium, amprolium, diclazuril, gallic acid and/or toltrazuril), but it does not cause side effects or drug resistance, and it may be safe to use for a long period of time as it comprises a natural product (mangosteen) with low toxicity.

In the present application, "prevention" means all acts that inhibit or delay development of disease by administration of the composition according to one embodiment, and "treatment" means all acts which improve or beneficially change symptoms of doubt and onset subjects of disease by administration of the composition according to one embodiment, and "alleviation" may mean all acts which reduce parameters associated with the condition where disease is treated by administration of the composition according to one embodiment, for example, at least the degree of symptoms. The disease may mean coccidiosis.

One aspect may provide a feed composition for preventing or alleviating coccidiosis, comprising a mangosteen.

The composition according to one embodiment may comprise a mangosteen as an active ingredient showing anticoccidial activity, and the mangosteen may be a mangosteen fruit. Mangosteen (*Garcinia mangostana* L.) is an evergreen tall tree which is a dicotyledonous plant belonging to the Soapberry order, Staphyleaceae family, native to Malaysia, and bears a fruit that is slightly larger than a flat ping-pong ball. The mangosteen fruit contains a representative antioxidant component called xanthone, and therefore, it has been known to have excellent antioxidant powder, and it has been known to have been used as a folk medicine in Thialand and Southeast Asia for a long time to treat wounds such as inflammation or cuts, and the like.

According to one embodiment, the mangosteen may be a pericarp (果皮, epicarp of the mangosteen fruit), mangosteen flesh (果肉), or a combination thereof (pericarp and flesh), and according to one embodiment, the composition comprising a mangosteen pericarp may have excellent anticoccidial activity. The mangosteen pericarp is a part that is not normally ingested and is discarded, and the composition according to one embodiment utiltizing this has advantages in terms of reuse of insoluble resources, environmental preservation and/or economic feasibility.

The composition according to one embodiment may have excellent anticoccidial activity than an individual component comprised by the mangosteen (for example, xanthone, alpha-mangostin, Epicatechin, and/or Quercetin).

In one embodiment, the mangosteen may be at least one (for example, 1 or more kinds, 2 or more kinds, 3 or more kinds, or all) selected from the group consisting of mangosteen raw materials, dried materials, pulverized materials and extracts.

The mangosteen raw material may mean a mangosteen without processing (for example, drying, pulverizing and/or extracting).

The mangosteen dried material may be obtained by drying the ginkgo leaf raw materials, and may be obtained by a method such as drying under reduced pressure, vacuum drying, boiling drying, room temperature drying and/or freeze drying and the like.

The mangosteen pulverized material may be obtained by pulverizing and/or crushing the mangosteen raw material and/or the mangosteen dried material, and may be obtained using a pulverizer.

In the present application, "extract" means a preparation which is concentrated by squeezing an herbal medicine with an appropriate leaching solution and evaporating the leaching solution, and it may be a dried material obtained by drying an extraction solution obtained by extraction treatment, a diluted solution or concentrate of the extraction solution, a modulated material and/or a purified material thereof. The mangosteen raw material may be prepared by using a common extraction method, a separation and purification method known in the art. As the extraction method, specifically, a method such as boiling water extraction, hot water extraction, enfleurage extraction, reflux cooling extraction or ultrasonic extraction, and the like may be used.

In one embodiment, the mangosteen extract may be purchased as commercially available one, or be prepared by appropriately selecting an extraction method and an extraction solvent commonly known in the medical or food industry. For example, as the extraction method, the common extraction method may include solvent extraction method, ultrasonic extraction method, filtration method and/or reflux extraction method, and the like. In one embodiment, the mangosteen extract may be prepared by extracting with an extraction solvent or fractionating by adding a fractionation solvent into the extract prepared by extracting with an extraction solvent. For example, the mangosteen extract may be extracted by a solvent extraction method using an extraction solvent selected from the group consisting of water, linear or branched chain alcohols having 1 to 4 carbon atoms, propylene glycol, butylene glycol, glycerin, acetone, ethyl acetate, butyl acetate, chloroform, diethyl ether, dichloromethane, hexane and mixtures thereof. Specifically, the alcohol having 1 to 4 carbon atoms may be ethanol, methanol, isopropanol, propanol, butanol, and/or tert-butanol. It may be extracted by adding the extraction solvent as much as 1 fold to 10 folds to the dried mangosteen amount, and specifically, it may be extracted by adding 2 folds to 3 folds. The extraction temperature may be 30°C to 100°C, 50°C to 90°C, or 60°C to 80°C. In addition, the extraction time may be 10 hours to 48 hours, 12 hours to 30 hours, or 18 hours to 24 hours. In addition, it may be repeatedly extracted as the time of extraction of 1 time to 5 times, or 2 times or 4 times.

In one specific embodiment, the mangosteen may be a mangosteen pericarp extract, and for example, may be an ethanol extract of a mangosteen pericarp, and the ethanol may be 50 to 100%(v/v), 60 to 100%(v/v), 65 to 100%(v/v), 70 to 100%(v/v), 75 to 100%(v/v), 80 to 100%(v/v), 50 to 95%(v/v), 60 to 95%(v/v), 65 to 95%(v/v), 70 to 95%(v/v), 75 to 95%(v/v), 80 to 95%(v/v), 50 to 90%(v/v), 60 to 90%(v/v), 65 to 90%(v/v), 70 to 90%(v/v), 75 to 90%(v/v), 80 to 90%(v/v), 50 to 85%(v/v), 60 to 85%(v/v), 65 to 85%(v/v), 70 to 85%(v/v), 75 to 85%(v/v), 80 to 85%(v/v), 50 to 80%(v/v), 60 to 80%(v/v), 65 to 80%(v/v), 70 to 80%(v/v), 75 to 80%(v/v), 50 to 75%(v/v), 60 to 75%(v/v), 65 to 75%(v/v), or 70 to 75%(v/v).

In the present application, "coccidiosis" is a disease in which a coccidium protozoan (protozoan which can induce coccidiosis, for example, *Eimeria* sp. coccidium protozoan) parasitizes in the cytoplasm of submucosal tissue in the epithelium of the digestive tract and destroys the epithelium and cause enteritis, and is a protozoal disease that causes economic damage due to weight gain degradation by soft stool, diarrhea and bloody stool and prolongation of the shipping age in a broiler farm. Coccidiosis may be expressed not only in broilers but also in birds and mammals, and specifically, the coccidiosis may infect cattle, rabbits, goats, dogs, cats, mice which are experimental animals, and rats, and the like, and in particular, it may cause fatal damage to poultry such as chickens and the like. In one embodiment, the coccidiosis may include acute coccidiosis, subacute coccidiosis, and chronic coccidiosis and the like. The acute coccidiosis may exhibit bloody stool, energy loss and anemia within 48 hours after infection, and the infected subject may die, and the subacute coccidiosis may exhibit bloody diarrhea and/or anemia symptoms after infection, and the chronic coccidiosis may exhibit symptoms of soft stool and/or body weight loss after diarrhea for 1~2 days after infection.

When an oocyst (cyst) of the coccidium protozoan species matures into a sporulated oocyst at high humidity and temperature, it is infectious, and when the oocyst is excreted in feces after passing through a certain life cycle in a body of a subject, it spreads easily and the life cycle of the oocyst is repeated. It is known that the oocytes (cyst) of the coccidium protozoan species are highly resistant to the external environment, and the cyst wall consists of two layers inside and outside. The outer layer of the cyst wall is a gelatin material that strongly resists external physical pressure and the inner layer is rich in nuclear proteins, so it may strongly resist chemical stimuli, for example, disinfectants. The oocyst of the coccidium protozoan species may comprise 4 sporocysts, and the sporocysts may each comprise 2 sporozoites, and they may be released in a form of sporocyst and sporozoite after infection to an animal in an oocyst form, and proliferate in cells, and the sporozoites that have undergone sexual reproduction and/or asexual reproduction may form an oocyst and be extracted in feces. In one embodiment, the sporozoite may be used in the same meaning as a protozoan, and the sporozoite (protozoan) may cause lesions.

According to one embodiment, the coccidiosis may be caused by an *Eimeria* sp. protozoan. In one embodiment, the *Eimeria* sp. protozoan may be at least one selected from the group consisting of *Eimeria acervulina, Eimeria tenella, Eimeria maxima, Eimeria necatrix, Eimeria brunetti, Eimeria hagani, Eimeria mitis, Eimeria praecox, Eimeria mivati, Eimeria aurati, Eimeria baueri, Eimeria lepidosirenis, Eimeria leucisci, Eimeria rutile, Eimeria vanasi, Eimeria amphisbaeniarum, Eimeria witchery, Eimeria yemenensae, Eimeria adenoeides, Eimeria colchici, Eimeria curvata, Eimeria dispersa, Eimeria duodenalis, Eimeria fraterculae, Eimeria gallopavonis, Eimeria innocua, Eimeria meleagridis, Eimeria meleagrimitis, Eimeria phasiani, Eimeria procera, Eimeria purpureicephali, Eimeria ahsata, Eimeria alabamensis, Eimeria alijevi, Eimeria aspheronica, Eimeria arloingi, Eimeria arundeli, Eimeria bakuensis, Eimeria bovis, Eimeria cameli, Eimeria caprina, Eimeria caprovina, Eimeria christenseni, Eimeria clethrionomyis, Eimeria coecicola, Eimeria contorta, Eimeria couesii, Eimeria crandallis, Eimeria dammahensis, Eimeria dowleri, Eimeria exigua, Eimeria falciformis, Eimeria farasanii, Eimeria ferrisi, Eimeria flavescens, Eimeria gallatii, Eimeria granulosa, Eimeria hirci, Eimeria intestinalis, Eimeria irresidua, Eimeria intricata, Eimeria jolchijevi, Eimeria krijgsmanni, Eimeria larimerensis, Eimeria macusaniensis, Eimeria magna, Eimeria marconii, Eimeria media, Eimeria melanuri, Eimeria myoxi, Eimeria nagpurensis, Eimeria nieschulzi, Eimeria ninakohlyakimovae, Eimeria ovinoidalis, Eimeria pallida, Eimeria palustris, Eimeria papillata, Eimeria perforans, Eimeria phocae, Eimeria pileata, Eimeria pipistrellus, Eimeria piriformis, Eimeria prionotemni, Eimeria procyonis, Eimeria punctate, Eimeria roobroucki, Eimeria saudiensis, Eimeria sealanderi, Eimeria separata, Eimeria stiedae, Eimeria ursini, Eimeria vermiformis, Eimeria weybridgensis, Eimeria wobati,* and *Eimeria zuernii.*

The composition according to one embodiment may have an excellent effect of prevention, alleviation and/or treatment of coccidiosis caused by at least one (for example, 1 kind or more, 2 kinds or more, 3 kinds or more, or 4 kinds or more) protozoan selected from the group consisting of *Eimeria* sp. protozoa described in Table 1 below, and the *Eimeria* sp. protozoa described in Table 1 below may cause coccidiosis of animals described in Table 1, respectively.

[Table 1]

|   | species | Host animal |
|---|---|---|
| 1 | *Eimeria acervulina* | Chicken (*Gallus gallus domesticus*) |
| 2 | *Eimeria tenella* | Chicken (*Gallus gallus domesticus*) |
| 3 | *Eimeria maxima* | Chicken (*Gallus gallus domesticus*) |
| 4 | *Eimeria necatrix* | Chicken (*Gallus gallus domesticus*) |
| 5 | *Eimeria brunetti* | Chicken (*Gallus gallus domesticus*) |
| 6 | *Eimeria hagani* | Chicken (*Gallus gallus domesticus*) |
| 7 | *Eimeria mitis* | Chicken (*Gallus gallus domesticus*) |
| 8 | *Eimeria praecox* | Chicken (*Gallus gallus domesticus*) |
| 9 | *Eimeria mivati* | Chicken (*Gallus gallus domesticus*) |
| 10 | *Eimeria aurati* | Goldfish (*Carassius auratus*) |
| 11 | *Eimeria baueri* | Carp (crucian carp (*Carassius carassius*)) |
| 12 | *Eimeria lepidosirenis* | South American lungfish (*Lepidosiren paradoxa*) |
| 13 | *Eimeria leucisci* | Barbel (common barbel (*Barbus barbus bocagei*)) |
| 14 | *Eimeria rutile* | European chub (*Leuciscus cephalus cabeda*), Iberian nase (*Chondrostoma polylepis polylepis*) |
| 15 | *Eimeria vanasi* | blue tilapia (*Oreochromis aureus*) |
| 16 | *Eimeria amphisbaeniarum* | Worm lizard (Mann's worm lizard (*Amphisbaena manni*)) |
| 17 | *Eimeria witchery* | Worm lizard (Mann's worm lizard (*A. manni*)) |

(continued)

|  | species | Host animal |
|---|---|---|
| 18 | *Eimeria yemenensae* | Rainbow agama (rock agama (*Agama yemenensis*)) |
| 19 | *Eimeria adenoeides* | Turkey (*Meleagris gallopavo*) |
| 20 | *Eimeria colchici* | Pheasant (common pheasant (*Phasianus colchicus*)) |
| 21 | *Eimeria curvata* | Ruddy ground dove (*Columbina talpacoti*), scaled dove (*Scardafella squammata*) |
| 22 | *Eimeria dispersa* | Turkey (*M. gallopavo*), quail (bobwhite quail (*Colinus virginianus*)) |
| 23 | *Eimeria duodenalis* | Pheasant (common pheasant (*Phasianus colchicus*)) |
| 24 | *Eimeria fraterculae* | Atlantic puffin (*Fratercula arctica*) |
| 25 | *Eimeria gallopavonis* | Turkey (*M. gallopavo*) |
| 26 | *Eimeria innocua* | Turkey (*M. gallopavo*) |
| 27 | *Eimeria meleagridis* | Turkey (*M. gallopavo*) |
| 28 | *Eimeria meleagrimitis* | Turkey (*M. gallopavo*) |
| 29 | *Eimeria phasiani* | Pheasant (*P. colchicus*) |
| 30 | *Eimeria procera* | Grey partridges (*Perdix perdix*) |
| 31 | *Eimeria purpureicephali* | Red-capped parrot (*Purpureicephalus spurius*) |
| 32 | *Eimeria ahsata* | Goat (*Capra hircus*), sheep (*Ovis aries*) |
| 33 | *Eimeria alabamensis* | Cattle (*Bos taurus*) |
| 34 | *Eimeria alijevi* | Goat (*C. hircus*) |
| 35 | *Eimeria aspheronica* | Goat (*C. hircus*) |
| 36 | *Eimeria arloingi* | Goat (*C. hircus*) |
| 37 | *Eimeria arundeli* | Common wombat (*Vombatus ursinus*) |
| 38 | *Eimeria bakuensis* | Sheep (*O. aries*) |
| 39 | *Eimeria bovis* | Cattle (*B. taurus*) |
| 40 | *Eimeria cameli* | Camels (*Camelus bactrianus*, *Camelus dromedarius*) |
| 41 | *Eimeria caprina* | Goat (*C. hircus*) |
| 42 | *Eimeria caprovina* | Goat (*C. hircus*) |
| 43 | *Eimeria christenseni* | Goat (*C. hircus*) |
| 44 | *Eimeria clethrionomyis* | Red-backed vole (*Clethrionomys gapperi*) |
| 45 | *Eimeria coecicola* | Rabbit (*Oryctolagus cuniculus*) |
| 46 | *Eimeria contorta* | Mouse (*Mus musculus*) |
| 47 | *Eimeria couesii* | Rice rat (*Oryzomys couesi*) |
| 48 | *Eimeria crandallis* | Sheep (*O. aries*) |
| 49 | *Eimeria dammahensis* | Scimitar-homed oryx (*Oryx dammah*) |
| 50 | *Eimeria dowleri* | Eastern red bat (*Lasiurus borealis*) |
| 51 | *Eimeria exigua* | Rabbit (*O. cuniculus*) |
| 52 | *Eimeria falciformis* | Mouse (*M. musculus*) |
| 53 | *Eimeria farasanii* | Mountain gazelle (*Gazella gazelle, farasani*) |
| 54 | *Eimeria ferrisi* | Mouse (*M. musculus*) |

(continued)

|  | species | Host animal |
|---|---|---|
| 55 | *Eimeria flavescens* | Rabbit (*O. cuniculus*) |
| 56 | *Eimeria gallatii* | Red-backed vole (*Clethrionomys gapperi*) |
| 57 | *Eimeria granulosa* | Goat (*C. hircus*) |
| 58 | *Eimeria hirci* | Goat (*C. hircus*) |
| 59 | *Eimeria intestinalis* | Rabbit (*O. cuniculus*) |
| 60 | *Eimeria irresidua* | Rabbit (*O. cuniculus*) |
| 61 | *Eimeria intricata* | Goat (*C. hircus*) |
| 62 | *Eimeria jolchijevi* | Goat (*C. hircus*) |
| 63 | *Eimeria krijgsmanni* | Mouse (*M. musculus*) |
| 64 | *Eimeria larimerensis* | Uinta ground squirrel (*Spermophilus armatus*) |
| 65 | *Eimeria macusaniensis* | Llamas (*Lama glama*), guanacos (*Lama guanicoe*), alpacas (*Vicugna pacos*), vicunas (*Vicugna vicugna*) |
| 66 | *Eimeria magna* | Rabbit (*O. cuniculus*) |
| 67 | *Eimeria marconii* | Red-backed vole (*Clethrionomys gapperi*) |
| 68 | *Eimeria media* | Rabbit (*O. cuniculus*) |
| 69 | *Eimeria melanuri* | Garden dormouse (*Eliomys quercinus*) |
| 70 | *Eimeria myoxi* | Garden dormouse (*Eliomys quercinus*) |
| 71 | *Eimeria nagpurensis* | Rabbit (*O. cuniculus*) |
| 72 | *Eimeria nieschulzi* | Brown rat (*R. norvegicus*) |
| 73 | *Eimeria ninakohlyakimovae* | Goat (*C. hircus*) |
| 74 | *Eimeria ovinoidalis* | Sheep (*O. aries*) |
| 75 | *Eimeria pallida* | Goat (*C. hircus*) |
| 76 | *Eimeria palustris* | Rice rat (marsh rice rat (*Oryzomys palustris*)) |
| 77 | *Eimeria papillata* | Mouse (*M. musculus*) |
| 78 | *Eimeria perforans* | Rabbit (*O. cuniculus*) |
| 79 | *Eimeria phocae* | Sable Island harbour seals (*Phoca vitulina*) |
| 80 | *Eimeria pileata* | Red-backed vole (*Clethrionomys gapperi*) |
| 81 | *Eimeria pipistrellus* | Kuhl's pipistrelle (*Pipistrellus kuhlii*) |
| 82 | *Eimeria piriformis* | Rabbit (*O. cuniculus*) |
| 83 | *Eimeria prionotemni* | Bennett's wallaby (*Macropus rufogriseus*) |
| 84 | *Eimeria procyonis* | Raccoon (*Procyon lotor*) |
| 85 | *Eimeria punctate* | Goat (C. *hircus*) |
| 86 | *Eimeria roobroucki* | Rabbit (*O. cuniculus*) |
| 87 | *Eimeria saudiensis* | Arabian oryx (*Oryx leucoryx*) |
| 88 | *Eimeria sealanderi* | Eastern red bat (*Lasiurus borealis*) |
| 89 | *Eimeria separata* | Mouse (*M. musculus*), rat (*Rattus rattus*) |
| 90 | *Eimeria stiedae* | Rabbit (*O. cuniculus*) |
| 91 | *Eimeria ursini* | Southern hairy nosed wombat (*Lasiorhinus latifrons*) |

(continued)

| | species | Host animal |
|---|---|---|
| 92 | *Eimeria vermiformis* | Mouse *(M. musculus)* |
| 93 | *Eimeria weybridgensis* | Sheep (O. *aries*) |
| 94 | *Eimeria wobati* | Southern hairy-nosed wombat (*L. latifrons*) |
| 95 | *Eimeria zuernii* | Cattle (*Bos taurus*) |

The composition according to one embodiment may have an excellent effect for prevention, alleviation and/or treatment of coccidiosis caused by *Eimeria tenella, Eimeria acervulina,* and/or *Eimeria maxima.*

In one embodiment, the prevention or alleviation of coccidiosis may mean at least one (for example, any one, 2 or more kinds, 3 or more kinds, or all) selected from the group consisting of the following (1) to (4), and for example, at least one selected from the group consisting of the following (1) to (4) may be reduce, inhibited and/or increased compared to a control group (negative control group and/or positive control group):

(1) reduction of at least one selected from the group consisting of lesion score (for example, appendix lesion score), fecal oocyst excretion amount and mortality;
(2) inhibition of weight loss due to coccidiosis;
(3) increase of an anticoccidial index (ACI); and
(4) reduction of cell invasion of an *Eimeria* sp. protozoan, propagation of the protozoan in cells, or both.

In one embodiment, the lesion scoring method which determines the lesion score may be performed by referring to Johnson JK & Reid WM (1970) document (Joyce Johnson, W.Malcolm Reid, Anticoccidial drugs: Lesion scoring techniques in battery and floor-pen experiments with chickens, Experimental parasitology, 1970), and the lesion score may be 0 to 4 degrees. In one embodiment, the lesion score may mean a lesion score measured in appendix, duodenum and/or jejunum, and it may be calculated by a sum of each of lesion scores measured in each organ (appendix, duodenum and/or jejunum).

In one embodiment, the fecal oocyst extraction amount may be measured using a microscope or using a counting chamber (for example, McMaster chamber) and the like by collecting feces excreted from a subject.

In one embodiment, the mortality may mean mortality of an animal subject in which coccidiosis is induced, and by performing a post mortem, the number of subjects died from causes other than coccidiosis may be excluded.

In one embodiment, the subject in which coccidiosis is induced may have a reduced body weight than a subject in which coccidiosis is not induced, and the composition according to one embodiment may inhibit weight loss by induction of coccidiosis.

In one embodiment, the anticoccidial index may be calculated by Equation 1 below, and in Equation 1, the lesion score may be calculated as aforementioned.

(Equation 1)

Anticoccidial index (ACI) = (survival rate after challenge inoculation (%)) + (daily weight gain compared to negative control group (%)) - (lesion score × 10) - (fecal oocyst excretion amount index)

The challenge inoculation may mean administration (for example, oral inoculation, etc.) of a protozoan capable of inducing coccidiosis. In one embodiment, the survival rate may be a survival rate measured at Day 5 to 10, Day 7 to 10, Day 8 to 10, Day 7 to 9, Day 7 to 8, or Day 7 after challenge inoculation, and by performing a post mortem, the survival rate may be measured by excluding the number of subjects died from causes other than coccidiosis.

The weight gain compared to the negative control group in Equation 1 above may be a value calculated by a percentage calculated on the basis of a value of the negative control group (for example, negative control group in which a protozoan is non-infected).

The lesion score in Equation 1 above is as described above.

In Equation 1 above, the fecal oocyst excretion amount index may be a numerical value of 0 when the calculated result value is at a level of 0% or more to less than 1%, 5 when the calculated result value is at a level of 1% or more to less than 26%, 10 when the calculated result value is at a level of 26% or more to less than 51%, 20 when the calculated result value is at a level of 51% or more to less than 76% and 40 when the calculated result value is at a level of 76% or more to 100% or less, by calculating a percentage based on a value of a negative control group (for example, negative control group in which a protozoan is infected).

In one embodiment, the mangosteen may be comprised in the feed composition by 1w/w% or less, less than 1w/w%, $10^{-1}$ w/w% or less, $5 \times 10^{-2}$ w/w% or less, $2.5 \times 10^{-2}$ w/w% or less, $2 \times 10^{-2}$ w/w% or less, $1.25 \times 10^{-2}$ w/w% or less, $10^{-2}$ w/w% or less, $9 \times 10^{-3}$ w/w% or less, $8 \times 10^{-3}$ w/w% or less, $7 \times 10^{-3}$ w/w% or less, $6 \times 10^{-3}$ w/w% or less, $5 \times 10^{-3}$ w/w% or less, $4 \times 10^{-3}$ w/w% or less, $10^{-7}$ w/w% or more, $10^{-6}$ w/w% or more, $10^{-5}$ w/w% or more, $10^{-4}$ w/w% or more, $5 \times 10^{-4}$ w/w% or more, $10^{-3}$ w/w% or more, $1.5 \times 10^{-3}$ w/w% or more, $2 \times 10^{-3}$ w/w% or more, $3 \times 10^{-3}$ w/w% or more, $4 \times 10^{-3}$ w/w% or more, $5 \times 10^{-3}$ w/w% or more, $10^{-7}$ to 1w/w%, $10^{-7}$ to $10^{-1}$w/w%, $10^{-7}$ to $5 \times 10^{-2}$ w/w%, $10^{-7}$ to $10^{-2}$ w/w%, $10^{-7}$ to $5 \times 10^{-3}$ w/w%, $10^{-7}$ to $4 \times 10^{-3}$ w/w%, $10^{-7}$ to $10^{-3}$ w/w%, $10^{-7}$ to $5 \times 10^{-4}$ w/w%, $10^{-7}$ to $10^{-4}$ w/w%, $10^{-7}$ to $10^{-5}$ w/w%, $10^{-6}$ to 1w/w%, $10^{-6}$ to $10^{-1}$w/w%, $10^{-6}$ to $5 \times 10^{-2}$ w/w%, $10^{-6}$ to $10^{-2}$ w/w%, $10^{-6}$ to $5 \times 10^{-3}$ w/w%, $10^{-6}$ to $4 \times 10^{-3}$ w/w%, $10^{-6}$ to $10^{-3}$ w/w%, $10^{-6}$ to $5 \times 10^{-4}$ w/w%, $10^{-6}$ to $10^{-4}$ w/w%, $10^{-6}$ to $10^{-5}$ w/w%, $10^{-5}$ to 1w/w%, $10^{-5}$ to $10^{-1}$w/w%, $10^{-5}$ to $5 \times 10^{-2}$ w/w%, $10^{-5}$ to $10^{-2}$ w/w%, $10^{-5}$ to $5 \times 10^{-3}$ w/w%, $10^{-5}$ to $4 \times 10^{-3}$ w/w%, $10^{-5}$ to $10^{-3}$ w/w%, $10^{-5}$ to $5 \times 10^{-4}$ w/w%, $10^{-5}$ to $10^{-4}$ w/w%, $10^{-4}$ to 1w/w%, $10^{-4}$ to $10^{-1}$w/w%, $10^{-4}$ to $5 \times 10^{-2}$ w/w%, $10^{-4}$ to $10^{-2}$ w/w%, $10^{-4}$ to $5 \times 10^{-3}$ w/w%, $10^{-4}$ to $4 \times 10^{-3}$ w/w%, $10^{-4}$ to $10^{-3}$ w/w%, $10^{-4}$ to $5 \times 10^{-4}$ w/w%, $10^{-3}$ to 1w/w%, $10^{-3}$ to $10^{-1}$w/w%, $10^{-3}$ to $5 \times 10^{-2}$ w/w%, $10^{-3}$ to $10^{-2}$ w/w%, $10^{-3}$ to $5 \times 10^{-3}$ w/w%, $10^{-3}$ to $4 \times 10^{-3}$ w/w%, $10^{-3}$ to $2 \times 10^{-3}$ w/w%, or $10^{-3}$ to $1.5 \times 10^{-3}$ w/w%. In one embodiment, the feed composition may be a feed (for example, assorted feed and/or ingredient feed finally ingested by an animal) comprising the active ingredient in the range based on the total weight.

In one embodiment, the mangosteen (for example, mangosteen extract) may be comprised in the feed composition at a concentration of 1000ppm or less, 500ppm or less, 400ppm or less, 300ppm or less, 250ppm or less, 200ppm or less, 125ppm or less, less than 125ppm, 100ppm or less, 90ppm or less, 80ppm or less, 70ppm or less, 65ppm or less, 62.5ppm or less, 60ppm or less, 50ppm or less, 0. 1ppm or more, 1ppm or more, 5ppm or more, 10ppm or more, 15ppm or more, 20ppm or more, 30ppm or more, 40ppm or more, 50ppm or more, 62.5ppm or more, 0.1 to 1000ppm, 0.1 to 500ppm, 0.1 to 400ppm, 0.1 to 300ppm, 0.1 to 250ppm, 0.1 to 200ppm, 0.1 to 125ppm, 0.1 to 100 ppm, 0.1 to 90ppm, 0.1 to 80ppm, 0.1 to 70ppm, 0.1 to 65ppm, 0.1 to 60ppm, 0.1 to 50ppm, 1 to 1000ppm, 1 to 500ppm, 1 to 400ppm, 1 to 300ppm, 1 to 250ppm, 1 to 200ppm, 1 to 125ppm, 1 to 100 ppm, 1 to 90ppm, 1 to 80ppm, 1 to 70ppm, 1 to 65ppm, 1 to 60ppm, 1 to 50ppm, 5 to 1000ppm, 5 to 500ppm, 5 to 400ppm, 5 to 300ppm, 5 to 250ppm, 5 to 200ppm, 5 to 125ppm, 5 to 100 ppm, 5 to 90ppm, 5 to 80ppm, 5 to 70ppm, 5 to 65ppm, 5 to 60ppm, 5 to 50ppm, 10 to 1000ppm, 10 to 500ppm, 10 to 400ppm, 10 to 300ppm, 10 to 250ppm, 10 to 200ppm, 10 to 125ppm, 10 to 100 ppm, 10 to 90ppm, 10 to 80ppm, 10 to 70ppm, 10 to 65ppm, 10 to 60ppm, 10 to 50ppm, 20 to 1000ppm, 20 to 500ppm, 20 to 400ppm, 20 to 300ppm, 20 to 250ppm, 20 to 200ppm, 20 to 125ppm, 20 to 100 ppm, 20 to 90ppm, 20 to 80ppm, 20 to 70ppm, 20 to 65ppm, 20 to 60ppm, 20 to 50ppm, 30 to 1000ppm, 30 to 500ppm, 30 to 400ppm, 30 to 300ppm, 30 to 250ppm, 30 to 200ppm, 30 to 125ppm, 30 to 100 ppm, 30 to 90ppm, 30 to 80ppm, 30 to 70ppm, 30 to 65ppm, 30 to 60ppm, 30 to 50ppm, 40 to 1000ppm, 40 to 500ppm, 40 to 400ppm, 40 to 300ppm, 40 to 250ppm, 40 to 200ppm, 40 to 125ppm, 40 to 100 ppm, 40 to 90ppm, 40 to 80ppm, 40 to 70ppm, 40 to 65ppm, 40 to 60ppm, 40 to 50ppm, 50 to 1000ppm, 50 to 500ppm, 50 to 400ppm, 50 to 300ppm, 50 to 250ppm, 50 to 200ppm, 50 to 125ppm, 50 to 100 ppm, 50 to 90ppm, 50 to 80ppm, 50 to 70ppm, 50 to 65ppm, or 50 to 60ppm.

In one embodiment, when the mangosteen (for example, mangosteen extract) is comprised within the above range, compared to a case of comprising the mangosteen outside the above range, the anticoccidial activity may be excellent.

According to one embodiment, the mangosteen extract (for example, mangosteen pericarp extract) have (1) superior anticoccidial activity when treated at the same concentration and/or (2) superior safety due to low toxicity (for example, higher minimum concentration showing cytotoxicity) to each individual component comprised in the mangosteen extract (for example, xanthone, alpha-mangostin, Epicatechin, and/or Quercetin). The mangosteen (for example, mangosteen extract) according to one embodiment may safely show excellent anticoccidial activity even if the concentration comprised in the composition is higher than each individual component comprised in mangosteen and/or may not show separate side effects even when ingested for a long period of time.

In the present application, "feed" may mean any natural or artificial diet, one-meal meal or the like for an animal to eat, ingest and digest or suitable therefor or a component of the one-meal meal. In the feed composition according to one embodiment, a concentrated feed and/or a special feed may be further comprised. The concentrated feed is a by-product obtained by purifying seed fruits and grains including grains such as wheat, oats, corn and the like, and may be bran including rice bran, wheat bran, barley bran and the like, sesame cake which is a by-product obtained by drilling soybeans, fluid, sesame seeds, flax seeds, coco palms, and the like for oil, and residues such as residual starch, which is a main component of starch residue that is the remainder after removing starch from sweet potatoes and potatoes, fish soluble obtained by concentrating fresh liquid (液狀物) obtained from fish meal, fish waste and fish, animal feed such as dried whey in which whey that is the remainder when casein is produced from meat meal (肉粉), blood meal, feather meal, skim milk powder, milk to cheese, skim milk, and the like, yeast, chlorella and/or seaweed, and the like.

The feed composition according to one embodiment may mean a feed in a form which is finally ingested by an animal, a dietary supplement capable of being mixed to feed, and/or a feed additive. The dietary supplement is for example, a composition containing a preparation which provides a therapeutic agent or a digestive agent to an animal, and may mean a composition which is not a common source of calorie intake of a living body, that is, an energy source, but is ingested in addition to a normal animal feed. The feed additive refers to a substance added to feed for a purpose of various effects such as nutrient supplementation and weight loss prevention, improvement of digestibility of fiber in feed, oil quality improvement, reproductive disorder prevention and fertility rate enhancement, prevention of high temperature stress in summer and the like. In one embodiment, it may mean a substance which is added for a purpose of prevention, alleviation or treatment of coccidiosis.

In one embodiment, the feed composition may be a feed additive, and when the feed additive according to one embodiment is mixed to feed (for example, mixed feed and/or ingredient feed finally ingested to an animal), it may be added in a weight of 0.001% or more, 0.005% or more, 0.01% or more, 0.05% or more, 0.1% or more, 0.5% or more, 1% or less, 0.5% or less, 0.1% or less, 0.05% or less, 0.01% or less, 0.005% or less, 0.001 to 1%, 0.001 to 0.5%, 0.001 to 0.1%, 0.001 to 0.05%, 0.001 to 0.01%, 0.001 to 0.005%, 0.005 to 1%, 0.005 to 0.5%, 0.005 to 0. 1%, 0.005 to 0.05%, 0.005 to 0.01%, 0.01 to 1%, 0.01 to 0.5%, 0.01 to 0.1%, 0.01 to 0.05%, 0.05 to 1%, 0.05 to 0.5%, 0.05 to 0.1%, 0.1 to 1%, 0.1 to 0.5%, or 0.5 to 1% based on the total feed weight, and it may be mixed with feed raw materials, supplementary feed, adjuvants and/or other kinds of additives and the like other than the active ingredient according to one embodiment.

One aspect may provide a pharmaceutical composition for preventing or treating coccidiosis, comprising a mangosteen, and the mangosteen comprised in the pharmaceutical composition is as described above. the mangosteen comprised in a pharmaceutical composition is as described above.

The pharmaceutical composition according to one embodiment may comprise a mangosteen pericarp, mangosteen flesh, or a combination thereof (mangosteen pericarp and flesh).

The pharmaceutical composition according to one embodiment may comprise at least one (for example, 1 kind or more, 2 kinds or more, 3 kinds or more, or all of 4 kinds) selected from the group consisting of raw material, dried material, pulverized material and extract of the mangosteen.

The pharmaceutical composition according to one embodiment may comprise an extract of mangosteen pericarp (for example, ethanol extract of mangosteen pericarp)

The pharmaceutical composition according to one embodiment may be used as a single formulation, and may be used by preparing a mixed formulation by further comprising an authorized pharmaceutical composition known to have an effect of preventing or treating coccidiosis. It may be formulated into a pharmaceutical unit administration form by adding a pharmaceutically acceptable carrier, excipient or diluent.

In the present application, "pharmaceutically acceptable" means that it does not significantly stimulate an organism and does not inhibit the biological activity and properties of an administration active substance. The pharmaceutical composition comprising the pharmaceutically acceptable carrier according to one embodiment may have any one formulation selected from the group consisting of a tablet, pill, powder, a granule, a capsule, suspension, internal solution, emulsion, syrup, sterilized aqueous solution, non-aqueous solution, suspension, emulsion, a freeze-dried formulation and a suppository.

The pharmaceutical composition may be various oral or parenteral formulations. In case of formulation, it may be prepared using a commonly used filler, extender, binder, wetting agent, disintegrant, diluent such as a surfactant, or excipient.

Solid preparations for oral administration include tablets, pills, powder, granules, capsules and the like, and these solid preparations may be prepared by mixing at least one excipient, for example, starch, calcium carbonate, sucrose or lactose, gelatin and the like to one or more compounds. In addition, other than simple excipients, lubricants such as magnesium stearate, talc and the like may be used. Liquid preparations for oral administration include suspension, internal solution, emulsion, syrup and the like, and various excipients, for example, wetting agents, sweeteners, fragrances, preservatives and the like other than water and liquid paraffin which are commonly used simple diluents may be comprised.

Preparations for parenteral administration may include sterilized aqueous solution, non-aqueous solution, suspension, emulsion, a freeze-dried formulation and a suppository. As the non-aqueous solution and suspension, propylene glycol, polyethylene glycol, vegetable oil such as olive oil, injectable ester such as ethyl oleate, and the like may be used. As a base of the suppository, witepsol macrogol, tween 61, cacao butter, laurin butter, glycerogelatin and the like may be used.

In one embodiment, the pharmaceutical composition may be used by formulation into various forms such as oral formulations such as powder, granules, tablets, capsules, suspension, emulsion, syrup, aerosols, and the like, injectable formulations of sterilized injection solutions, and the like according to a common method for each purpose of use, and it may be orally administered or administered through various routes including intravenous, intraperitoneal, subcutaneous, intra-rectal, topical administration and the like.

In one embodiment, a carrier, excipient or diluent or the like may be further comprised in the pharmaceutical composition additionally, and the example of the suitable carrier, excipient or diluent which may be comprised may include lactose, dextrose, sucrose, sorbitol, mannitol, xylitol, erythritol, maltitol, starch, acacia gum, alginate, gelatin, calcium phosphate, calcium silicate, cellulose, methyl cellulose, amorphous cellulose, polyvinyl pyrrolidone, water, methylhydroxybenzoate, propylhydroxybenzoate, talc, magnesium stearate and mineral oil and the like. In addition, the pharmaceutical composition may further comprise a filler, anticoagulant, lubricant, wetting agent, flavoring, emulsifier, preservative or the like additionally.

In one embodiment, the effective amount of the active ingredient (mangosteen (for example, extract of mangosteen)) in the pharmaceutical composition may differ depending on the patient (subject)'s age, gender and body weight, and in general, 0.0001 to 0.001mg/kg, 0.0001 to 0.01mg/kg, 0.0001 to 0.1mg/kg, 0.0001 to 1mg/kg, 0.0001 to 10mg/kg, 0.0001 to 100mg/kg, 0.0001 to 231mg/kg, 0.0001 to 250mg/kg, 0.0001 to 500mg/kg, 0.0001 to 1000mg/kg, 0.001 to O.Olmg/kg, 0.001 to 0.1mg/kg, 0.001 to 1mg/kg, 0.001 to 10mg/kg, 0.001 to 100mg/kg, 0.001 to 231mg/kg, 0.001 to 250mg/kg, 0.001 to 500mg/kg, 0.001 to 1000mg/kg, 0.01 to 0.1mg/kg, 0.01 to 1mg/kg, 0.01 to 10mg/kg, 0.01 to 100mg/kg, 0.01 to 250mg/kg, 0.01 to 500mg/kg, 0.01 to 1000mg/kg, 0.1 to 1mg/kg, 0.1 to 10mg/kg, 0.1 to 100mg/kg, 0.1 to 231mg/kg, 0.1 to 250mg/kg, 0.1 to 500mg/kg, 0.1 to 1000mg/kg, 1 to 10mg/kg, 1 to 100mg/kg, 1 to 231mg/kg, 1 to 250mg/kg, 1 to 500mg/kg, 1 to 1000mg/kg, 10 to 100mg/kg, 10 to 231mg/kg, 10 to 250mg/kg, 10 to 500mg/kg, 10 to 1000mg/kg, 100 to 231mg/kg, 100 to 250mg/kg, 100 to 500mg/kg, 100 to 1000mg/kg, 250 to 500mg/kg, or 250 to 1000mg/kg per body weight kg may be administered daily or on alternate days or be administered by dividing into 1 to 3 times per day. However, since it may be increased or decreased according to the administration route, severity of disease, gender, body weight, age and the like, the dosage does not limit the scope of the present application in any way. In one embodiment, when the composition is administered intraperitoneally, it may be administered at a concentration of 0.001 to 250mg/kg or 0.001 to 231mg/kg.

In one embodiment, the dosage of the pharmaceutical composition may be in various ranges depending on the patient's body weight, age, gender, health status, diet, administration time, administration method, excretion rate and severity of disease and the like.

In one embodiment, the mangosteen may be comprised in the pharmaceutical composition by 1w/w% or less, less than 1w/w%, $10^{-1}$ w/w% or less, $5 \times 10^{-2}$ w/w% or less, $2.5 \times 10^{-2}$ w/w% or less, $2 \times 10^{-2}$ w/w% or less, $1.25 \times 10^{-2}$ w/w% or less, $10^{-2}$ w/w% or less, $9 \times 10^{-3}$ w/w% or less, $8 \times 10^{-3}$ w/w% or less, $7 \times 10^{-3}$ w/w% or less, $6 \times 10^{-3}$ w/w% or less, $5 \times 10^{-3}$ w/w% or less, $4 \times 10^{-3}$ w/w% or less, $10^{-7}$ w/w% or more, $10^{-6}$ w/w% or more, $10^{-5}$ w/w% or more, $10^{-4}$ w/w% or more, $5 \times 10^{-4}$ w/w% or more, $10^{-3}$ w/w% or more, $1.5 \times 10^{-3}$ w/w% or more, $2 \times 10^{-3}$ w/w% or more, $3 \times 10^{-3}$ w/w% or more, $4 \times 10^{-3}$ w/w% or more, $5 \times 10^{-3}$ w/w% or more, $10^{-7}$ to 1w/w%, $10^{-7}$ to $10^{-1}$w/w%, $10^{-7}$ to $5 \times 10^{-2}$ w/w%, $10^{-7}$ to $10^{-2}$ w/w%, $10^{-7}$ to $5 \times 10^{-3}$ w/w%, $10^{-7}$ to $4 \times 10^{-3}$ w/w%, $10^{-7}$ to $10^{-3}$ w/w%, $10^{-7}$ to $5 \times 10^{-4}$ w/w%, $10^{-7}$ to $10^{-4}$ w/w%, $10^{-7}$ to $10^{-5}$ w/w%, $10^{-6}$ to 1w/w%, $10^{-6}$ to $10^{-1}$w/w%, $10^{-6}$ to $5 \times 10^{-2}$ w/w%, $10^{-6}$ to $10^{-2}$ w/w%, $10^{-6}$ to $5 \times 10^{-3}$ w/w%, $10^{-6}$ to $4 \times 10^{-3}$ w/w%, $10^{-6}$ to $10^{-3}$ w/w%, $10^{-6}$ to $5 \times 10^{-4}$ w/w%, $10^{-6}$ to $10^{-4}$ w/w%, $10^{-6}$ to $10^{-5}$ w/w%, $10^{-5}$ to 1w/w%, $10^{-5}$ to $10^{-1}$w/w%, $10^{-5}$ to $5 \times 10^{-2}$ w/w%, $10^{-5}$ to $10^{-2}$ w/w%, $10^{-5}$ to $5 \times 10^{-3}$ w/w%, $10^{-5}$ to $4 \times 10^{-3}$ w/w%, $10^{-5}$ to $10^{-3}$ w/w%, $10^{-5}$ to $5 \times 10^{-4}$ w/w%, $10^{-5}$ to $10^{-4}$ w/w%, $10^{-4}$ to 1w/w%, $10^{-4}$ to $10^{-1}$w/w%, $10^{-4}$ to $5 \times 10^{-2}$ w/w%, $10^{-4}$ to $10^{-2}$ w/w%, $10^{-4}$ to $5 \times 10^{-3}$ w/w%, $10^{-4}$ to $4 \times 10^{-3}$ w/w%, $10^{-4}$ to $10^{-3}$ w/w%, $10^{-4}$ to $5 \times 10^{-4}$ w/w%, $10^{-3}$ to 1w/w%, $10^{-3}$ to $10^{-1}$w/w%, $10^{-3}$ to $5 \times 10^{-2}$ w/w%, $10^{-3}$ to $10^{-2}$ w/w%, $10^{-3}$ to $5 \times 10^{-3}$ w/w%, $10^{-3}$ to $4 \times 10^{-3}$ w/w%, $10^{-3}$ to $2 \times 10^{-3}$ w/w%, or $10^{-3}$ to $1.5 \times 10^{-3}$ w/w.

In one embodiment, the mangosteen (for example, mangosteen extract) may be comprised in the pharmaceutical composition at a concentration of 1000ppm or less, 500ppm or less, 400ppm or less, 300ppm or less, 250ppm or less, 200ppm or less, 125ppm or less, less than 125ppm, 100ppm or less, 90ppm or less, 80ppm or less, 70ppm or less, 65ppm or less, 60ppm or less, 62.5ppm or less, 50ppm or less, 0.1ppm or more, 1ppm or more, 5ppm or more, 10ppm or more, 15ppm or more, 20ppm or more, 30ppm or more, 40ppm or more, 50ppm or more, 62.5ppm or more, 0.1 to 1000ppm, 0.1 to 500ppm, 0.1 to 400ppm, 0.1 to 300ppm, 0.1 to 250ppm, 0.1 to 200ppm, 0.1 to 125ppm, 0.1 to 100 ppm, 0.1 to 90ppm, 0.1 to 80ppm, 0.1 to 70ppm, 0.1 to 65ppm, 0.1 to 60ppm, 0.1 to 50ppm, 1 to 1000ppm, 1 to 500ppm, 1 to 400ppm, 1 to 300ppm, 1 to 250ppm, 1 to 200ppm, 1 to 125ppm, 1 to 100 ppm, 1 to 90ppm, 1 to 80ppm, 1 to 70ppm, 1 to 65ppm, 1 to 60ppm, 1 to 50ppm, 5 to 1000ppm, 5 to 500ppm, 5 to 400ppm, 5 to 300ppm, 5 to 250ppm, 5 to 200ppm, 5 to 125ppm, 5 to 100 ppm, 5 to 90ppm, 5 to 80ppm, 5 to 70ppm, 5 to 65ppm, 5 to 60ppm, 5 to 50ppm, 10 to 1000ppm, 10 to 500ppm, 10 to 400ppm, 10 to 300ppm, 10 to 250ppm, 10 to 200ppm, 10 to 125ppm, 10 to 100 ppm, 10 to 90ppm, 10 to 80ppm, 10 to 70ppm, 10 to 65ppm, 10 to 60ppm, 10 to 50ppm, 20 to 1000ppm, 20 to 500ppm, 20 to 400ppm, 20 to 300ppm, 20 to 250ppm, 20 to 200ppm, 20 to 125ppm, 20 to 100 ppm, 20 to 90ppm, 20 to 80ppm, 20 to 70ppm, 20 to 65ppm, 20 to 60ppm, 20 to 50ppm, 30 to 1000ppm, 30 to 500ppm, 30 to 400ppm, 30 to 300ppm, 30 to 250ppm, 30 to 200ppm, 30 to 125ppm, 30 to 100 ppm, 30 to 90ppm, 30 to 80ppm, 30 to 70ppm, 30 to 65ppm, 30 to 60ppm, 30 to 50ppm, 40 to 1000ppm, 40 to 500ppm, 40 to 400ppm, 40 to 300ppm, 40 to 250ppm, 40 to 200ppm, 40 to 125ppm, 40 to 100 ppm, 40 to 90ppm, 40 to 80ppm, 40 to 70ppm, 40 to 65ppm, 40 to 60ppm, 40 to 50ppm, 50 to 1000ppm, 50 to 500ppm, 50 to 400ppm, 50 to 300ppm, 50 to 250ppm, 50 to 200ppm, 50 to 125ppm, 50 to 100 ppm, 50 to 90ppm, 50 to 80ppm, 50 to 70ppm, 50 to 65ppm, or 50 to 60ppm.

According to one embodiment, the mangosteen extract (for example, mangosteen extract and/or mangosteen pericarp extract) have (1) superior anticoccidial activity when treated at the same concentration and/or (2) superior safety due to low toxicity (for example, higher minimum concentration showing cytotoxicity) to each individual component comprised in the mangosteen extract (for example, xanthone, alpha-mangostin, Epicatechin, and/or Quercetin). The mangosteen (for example, mangosteen extract) according to one embodiment may safely show excellent anticoccidial activity even if the concentration comprised in the composition is higher than each individual component comprised in mangosteen and/or may not show separate side effects even when ingested for a long period of time.

In one embodiment, the pharmaceutical composition may be administered to a subject through various routes. The administration may mean providing a certain substance into a subject (patient) by any appropriate method, and the administration route of the pharmaceutical composition may be oral administration and/or parenteral administration through all common routes as long as to reach a target tissue. In case of parenteral administration, external application for skin, intraperitoneal injection, intra-rectal injection, subcutaneous injection, intravenous injection, intramuscular injection and/or intrathoracic injection may be selected. In addition, the composition according to one embodiment may be administered using any device capable of delivering the active ingredient into a target cell.

Other aspect may provide an antiprotozoal composition against an *Eimeria* sp. protozoan, comprising a mangosteen. Matters for the mangosteen and/or *Eimeria* sp. protozoan are described above.

The antiprotozoal composition according to one embodiment may comprise a mangosteen pericarp, a mangosteen flesh or a combinantion thereof (mangosteen pericarp and flesh).

The antiprotozoal composition according to one embodiment may comprise at least one (for example, 1 kind or more, 2 kinds or more, 3 kinds or more, or all the 4 kinds) selected from the group consisting of raw materials, dried materials, pulverized materials and extracts of the mangosteen.

The antiprotoxoal composition according to one specific embodiment, may comprise a flesh extract of the mangosteen (for example, mangosteen pericarp ethanol extract).

In one embodiment, that the antiprotozoal activity (effect, efficacy) against an *Eimeria* sp. protozoan is excellent may mean the following characteristics of (1) and/or (2), and for example, it may exhibit the following characteristics of (1) and/or (2) compared to a control group (negative control group and/or positive control group):

(1) Excellent effect of killing an *Eimeria* sp. protozoan; and/or
(2) inhibition of a cell invasion effect of an *Eimeria* sp. protozoan and/or a propagation effect of the protozoan in cells.

In one embodiment, mangosteen (for example, mangosteen extract) may be comprised in the antiprotozoal composition in the aforementioned concentration range in the feed composition and/or pharmaceutical composition. In one embodiment, the composition comprising the active ingredient within the aforementioned concentration range may have the excellent antiprotozoal activity compared to a case of comprising it in a range other than the concentration range.

Other aspect may provide a method for preventing, alleviating or treating coccidiosis, comprising a step of administering the composition (for example, the feed composition, the feed additive, the pharmaceutical composition and/or the antiprotozoal composition) to an animal. In one embodiment, before the administering the composition, confirming (selecting) a subject (patient) in need of preventing, alleviating or treating of coccidiosis may be further comprised. The composition and coccidiosis are as described above. According to one embodiment, the confirming a subject may comprise detecting an oocyst of a protozoan capable of inducing coccidium from feces separated from a subject.

In the method for preventing, alleviating or treating coccidiosis according to one embodiment, the administration method, administration route and/or dosage of the composition are as described above.

According to one embodiment, the composition may be administered in a pharmaceutically effective dose. In the present application, 'pharmaceutically effective dose' means an amount sufficient for treating disease at a reasonable benefit/danger ratio applicable to medical treatment, and the effective dose level may be determined according to the patient's disease type, severity, drug activity, sensitivity to a drug, administration time, administration route and excretion ratio, treatment period, an element comprising a drug used simultaneously and other elements well known in the medical field. According to one embodiment, the composition may be administered as an individual therapeutic agent or be administered in combination with other anticoccidial agents, and may be administered simultaneously, separately or sequentially with a conventional therapeutic agent, and may be administered single or multiple. Taking all of the elements into consideration, it is important to administer an amount that can obtain the maximum effect with a minimum amount without side effects, and this may be easily determined by those skilled in the art.

In one embodiment, the subject to which the method for preventing, alleviating or treating coccidiosis is applied means an animal in which coccidiosis is developed or may be developed, and the animal may be mammals including humans, horses, cattle, mice, rats, dogs, cats and the like, birds including poultry (for example, breeders, broilers and/or laying hens, etc.) and the like, fish, amphibians, and/or reptiles, and the like.

In one embodiment, the animal to which the method for preventing, alleviating or treating coccidiosis is applied may be at least one (for example, 1 kind or more, 2 kinds or more, or 3 kinds or more) selected from the group consisting of the animals described in Table 1 above, and for example, it may be at least one (for example, 1 kind or more, 2 kinds or more, or 3 kinds or more) selected from the group consisting of humans, chickens, ducks, geese, turkeys, quails, pheasants, pigeons, parrots, cattle, pigs, goats, sheep, horses, antelopes, oryxes, monkeys, cats, dogs, mice, rats, rabbits, racoons, squirrels, bats, guinea pigs, camels, llamas, alpaca, wombats, lizards, goldfish, crucian carp, tilapias, barbells, lungfish and European chubs. In one embodiment, the animal may be an animal except for human.

Other aspect provides a use of a mangosteen for the manufacture of a composition (for example, feed composition, pharmaceutical composition) for preventing, alleviating and treating coccidiosis or an antiprotozoal composition; a use of a mangosteen for using in prevention, alleviation and/or treatment of coccidiosis; and/or a use of a mangosteen for using in antiprotozoa against an *Eimeria* sp. protozoan (for example, *Eimeria* sp. protozoa killing; and/or cell invasion and/or propagation inhibition of *Eimeria* sp. protozoa). In the above use, a mangosteen is as described above, and for example, a mangosteen may be raw materials, dried materials, pulverized materials and extracts of the mangosteen. In the above use, coccidiosis, antiprotozoal and *Eimeria* sp. protozoa are as described above.

[ADVANTAGEOUS EFFECTS]

The composition comprising a mangosteen according to one embodiment has excellent direct killing effect on protozoa capable of inducing coccidiosis, cell invasion inhibitory effect of the protozoa, and/or propagation inhibitory effect of the protozoa in cells, and has excellent effects of preventing, alleviating and treating coccidiosis *in vivo.*

[BRIEF DESCRIPTION OF THE DRAWINGS]

FIG. 1 shows the cell invasion rate inhibitory effect of the protozoa according to treatment of the mangosteen, xanthone, and anticoccidial agents (diclazuril, salinomycin).
FIG. 2 shows the propagation inhibitory effect of the protozoa in cells according to treatment of the mangosteen, xanthone, and anticoccidial agents (diclazuril, salinomycin).
FIG. 3 shows the result of measuring the acid resistance of the mangosteen extract.
FIG. 4 shows the result of measuring the heat resistance of the mangosteen extract.

[MODE FOR INVENTION]

The present invention will be described in more detail by the following examples, but it is not intended to limit the scope by the following examples.

**Example *1. In vivo* anticoccidial activity of mangosteen**

**Example 1-1. Experimental facility**

An in vivo anticoccidial efficacy evaluation test was performed in an animal experiment facility in Gyeongsangnam-do, South Korea. One-day-old female Ross broilers were individually weighed and randomly divided into groups to use in an experiment. Matters and conditions for experimental design were described in Table 2.

[Table 2]

| Category | Experiment variable |
| --- | --- |
| Breeding type | Cage |
| Broiler stocking age | 1-day-old |
| Total experiment period | 22 days |
| Gender | Female |
| Number of broilers per cage | 20 |

(continued)

| Category | Experiment variable |
|---|---|
| Number of repetitions per treatment group | 2 repetitions |
| Number of treatment groups | 5 |
| Total number of broilers | 200 broilers |
| Kind of challenge inoculation protozoa | *Eimeria tenella* |
| Number of challenge inoculation protozoa | 10,000 oocysts oral inoculation/broiler |

A breeding farm was managed according to the Korean poultry breeding management guidelines. Cages and the breeding farm were cleaned and disinfected before starting the test. The temperature of 40 to 41°C and the humidity of 40 to 50% of the breeding farm were maintained, and it was continuously monitored.

**Example 1-2. Preparation of mangosteen extract**

Raw powder 120kg of drying and pulverizing a mangosteen (*Garcinia mangostana* L. source: Heung-ill herb. Co.,Ltd; country of origin: China, Changsha, Changsha herbway biotech of Wangcheng district. Co.,Ltd; purchased (acquired) on June, 2020) pericarp was extracted twice by immersing in a constant-temperature water bath using 75% ethanol (grain alcohol, purified ethyl alcohol) as a solvent. After extraction, it was filtered using filter paper, and then concentrated under reduced pressure using a rotary decompression concentrator, and powdered to finally obtain about 1kg of extracted powder, and it was used in the subsequent experiment.

**Example 1-3. Experimental design**

For the feed, Korea Feed A1-choi product was used, and each material (salinomycin (Cheil Bio Cheilsalino-60 product), xanthone (Sigma Aldrich X600 product), and the mangosteen extract prepared in Example 1-2 above) was added to the feed, respectively, at a concentration described in Table 3 below to be self-mixed. Antibiotics and supplements were not used in general feed and mixed feed, and no anticoccidial agent other than each material was added. Broilers were fed ad libitum throughout the experiment period. After putting 20 1-day-age broilers in cages randomly placed for each of the control group or test group and breeding, general feed was fed for 7 days, and then the mixed feed prepared above was ingested by dividing by control group or test group.

The feed formulation administered to the control group (negative control group or positive control group) and test group and whether coccidiosis was induced by *Eimeria tenella* were described in Table 3 below. 20 1-day-old broilers were put in a cage randomly arranged for each test group, and after 7 days of feeding the general feed, the formulated feed as described in Table 3 was divided into each test group and ingested.

14-day-old broilers were orally inoculated with 10,000 oocysts (90% sporulated Eimeria tenella oocysts) per individual to induce coccidiosis.

[Table 3]

| Group | Treatment |
|---|---|
| Non-infected negative control group | General feed |
| Infected negative control group | *Eimeria tenella* infection + general feed |
| Positive control group 1 (salinomycin treated group) | *Eimeria tenella* infection + formulated feed comprising salinomycin 60ppm |
| Xanthone treated group | *Eimeria tenella* infection + formulated feed comprising xanthone 62.5ppm |
| Mangosteen extract treated group | *Eimeria tenella* infection + formulated feed comprising mangosteen extract 62.5ppm |

**Example 1-4. Measurement of anticoccidial activity of mangosteen**

The anticoccidial efficacy by test group designed in Example 1-3 above was shown as an anticoccidial index

14

(ACI), and the anticoccidial index was calculated by Equation 2 below. The ACI score is out of 200 points, and the higher the ACI score, the more excellent the anticoccidial ability, and when it is 120 points or more to less than 140 points, it is determined that it is effective as an anticoccidial material, and when it is 140 points or more to less than 160 points, it is determined that it is excellent as an anticoccidial material, and when it is 160 points or more, it is determined that the anticoccidial effect is very excellent. (Luis Miguel De Pablos et al., Anticoccidial activity of maslinic acid against infection with Eimeria tenella in chickens, Parasitol Res, 2010)

(Equation 2)

Anticoccidial index (ACI) = (survival rate after challenge inoculation (%)) + (daily weight gain compared to negative control group (RWG, %)) - (lesion score × 10) - (fecal oocyst excretion amount index)

1) Survival rate: the number of dead individuals was recorded daily, and post mortem autopsy was performed to determine a cause of mortality, and the number of individuals who died due to causes other than coccidiosis was excluded. The survival rate (%) up to the 8th day after challenge inoculation was used for anticoccidial index calculation.

2) Daily weight gain: body weight was measured for each cage before challenge inoculation with a protozoan into an individual and at the 7th day after challenge inoculation, and the difference was divided by the number of days to calculate the daily weight gain (ADG, g/d). 'Daily weight gain compared to the negative control group (RWG, %)'which was calculated by dividing the daily weight gain (ADG, average daily gain; g/d) of each experimental group by the weight gain (ADG, g/d) of the non-infected negative control group and multiplying by 100 was used for anticoccidial index calculation.

The daily weight gain (ADG, g/d) measured in each control group and test group and the daily weight gain compared to the negative control group (RWG, %) were shown in Table 4 below.

[Table 4]

| Treatment group | Initial body weight (7-day-old) | Intermediate body weight (14-day-old, before challenge inoculation) | End body weight (21-day-old, on the 7th day of challenge inoculation) | Day 14 ~ Day 21 ADG | RWG |
|---|---|---|---|---|---|
| Non-infected negative control group | 197.1 | 473.7 | 956.8 | 65.99 | 100 |
| Infected negative control group | 200.1 | 493.2 | 886.9 | 53.98 | 82 |
| Salinomycin treated group | 192.9 | 455.9 | 917.6 | 62.46 | 94 |
| Xanthone treated group | 189.8 | 470.9 | 887.0 | 56.42 | 85 |
| Mangosteen extract treated group | 200.8 | 506.5 | 973.9 | 65.51 | 99 |

3) Lesion scoring: On the 8th day after challenge inoculation, an autopsy was conducted for 4 broilers per cage, and the intestines were incised and opened. Scoring was performed for each coccidial lesion in the appendix region of the broilers. The lesion scoring method was performed by referring to Johnson JK & Reid WM (1970) document (Joyce Johnson, W.Malcolm Reid, Anticoccidial drugs: Lesion scoring techniques in battery and floor-pen experiments with chickens, Experimental parasitology, 1970). The lesion score is on a scale of 0-4, and 0 point corresponds to normal appendix, and 1 point is a mild infection symptom, and 2 points is a moderate infection symptom, and 3 points is a severe infection symptom and 4 points is a case of showing a very severe infection symptom or causing death. The appendix lesion score measured in each control group and test group was described in Table 5 below. The measured appendix lesion score was used for calculation of the anticoccidial index by multiplying by 10.

[Table 5]

| Group | Appendix lesion score |
|---|---|
| Non-infected negative control group | 0 |
| Infected negative control group | 3.1 |
| Salinomycin treated group | 2.7 |
| Xanthone treated group | 2.4 |
| Mangosteen extract treated group | 1.9 |

4) Fecal oocyst excretion amount: the entire feces on the 6 ~ 8th day of challenge inoculation were collected by cage, mixed evenly, and then randomly sampled 3 times in total by 1g each. After floating the oocysts in 1g feces using salt water, the oocyst excretion amount was measured using McMaster chamber, and the result was described in Table 6 below.

[Table 6]

| Group | Oocyst excretion amount/gram |
|---|---|
| Non-infected negative control group | 0.00E+00 |
| Infected negative control group | 1.90E+08 |
| Salinomycin treated group | 1.30E+08 |
| Xanthone treated group | 1.80E+08 |
| Mangosteen extract treated group | 1.30E+08 |

The oocyst excretion amount (%) compared to the infected negative control group was calculated by dividing the oocyst excretion amount by each group into the oocyst excretion amount of the infected negative control group and multiplying by 100. The oocyst excretion amount index was calculated as 0 when the calculated oocyst excretion amount compared to the infected negative control group was at a level of 0% to less than 1%, 5 when it was 1% or more to less than 26%, 10 when it was 26% or more to less than 51%, 20 when it was 51% or more to less than 76%, and 40 when it was 76% or more to 100% or less, and this was used for anticoccidial index calculation.

As aforementioned, the anticoccidial index by each test group measured by Equation 2 above was shown in Table 7 below. As shown in Table 7, it could be seen that the anticoccidial index in the mangosteen extract treated group was higher than the positive control group, salinomycin treated groups, so it showed the excellent anticoccidial efficacy.

[Table 7]

| Group | Anticoccidial index (ACI) |
|---|---|
| Non-infected negative control group | 197 |
| Infected negative control group | 101 |
| Salinomycin treated group | 141 |
| Xanthone treated group | 122 |
| Mangosteen extract treated group | 151 |

**Example 2. Direct killing effect against *Eimeria* sp. protozoan of mangosteen**

**Example 2-1. Measurement of protozoal direct killing effect of mangosteen**

In the present example, protozoan (sporozoite) direct killing ability evaluation was conducted against 3 representative Eimeria kinds known to be infected in most farms (*E. tenella, E. acervulina, E. maxima*).

A certain amount of oocysts of each protozoan was put in a tube containing glass beads and pulverized, and then to remove the crushed oocyst cell wall and other debris, internal sporocysts were purified using the percoll density gradient, and washed with PBS solution. A reagent comprising sodium taurocholic acid (Sigma aldrich, USA) and trypsin (Gibco, USA), respectively, was treated to sporocysts of *Eimeria tenella, Eimeria acervulina* and *Eimeria maxima* for excystation and they were incubated, and then they were washed with PBS solution once and protozoa were obtained.

After reacting the mangosteen pericarp extract, epicatechin, alpha-mangostin, xanthone and anticoccidial agents, salinomycin and diclazuril (hereinafter, material) with 3 kinds of *Eimeria* protozoa at various concentrations of 1 to 500ppm, respectively, only alive protozoa (sporozoites) were counted through microscopic observation. Then, the death rate (%) of the protozoa when each material was treated compared to the PBS-treated negative control group was calculated and shown in Table 8 to Table 10 and Table 12, respectively, and the minimum concentration which directly killed the protozoa by 100% was shown in Table 11 below.

[Table 8]

| *Eimeria acervulina* protozoal death rate (%) | | | | |
|---|---|---|---|---|
| ppm | Diclazuril | Salinomycin | Xanthone | Mangosteen pericarp extract |
| 500 | 44 | 100 | 25 | 100 |
| 250 | 12 | 100 | 11 | 100 |
| 125 | 23 | 73 | 0 | 100 |
| 100 | 0 | 60 | 0 | 100 |
| 50 | 0 | 0 | 0 | 100 |
| 10 | 0 | 0 | 0 | 91 |
| 5 | 0 | 0 | 0 | 65 |
| 1 | 0 | 0 | 0 | 16 |

[Table 9]

| *Eimeria tenella* protozoal death rate (%) | | | | |
|---|---|---|---|---|
| ppm | Diclazuril | Salinomycin | Xanthone | Mangosteen pericarp extract |
| 500 | 44 | 100 | 15 | 100 |
| 250 | 35 | 100 | 0 | 100 |
| 125 | 0 | 74 | 10 | 100 |
| 100 | 0 | 46 | 0 | 100 |
| 50 | 0 | 0 | 0 | 100 |
| 10 | 0 | 0 | 0 | 100 |
| 5 | 0 | 0 | 0 | 83 |
| 1 | 0 | 0 | 0 | 59 |

[Table 10]

| *Eimeria maxima* protozoal death rate (%) | | | | |
|---|---|---|---|---|
| ppm | Diclazuril | Salinomycin | Xanthone | Mangosteen pericarp extract |
| 500 | 30 | 100 | 12 | 100 |
| 250 | 16 | 97 | 5 | 100 |
| 125 | 0 | 65 | 0 | 100 |

(continued)

| Eimeria maxima protozoal death rate (%) | | | | |
|---|---|---|---|---|
| ppm | Diclazuril | Salinomycin | Xanthone | Mangosteen pericarp extract |
| 100 | 0 | 59 | 0 | 100 |
| 50 | 0 | 0 | 0 | 100 |
| 10 | 0 | 0 | 0 | 84 |
| 5 | 0 | 0 | 0 | 48 |
| 1 | 0 | 0 | 0 | 17 |

[Table 11]

| Treatment group | Minimum concentration for killing 100% protozoa (ppm) | | |
|---|---|---|---|
| | Eimeria tenella | Eimeria acervulina | Eimeria maxima |
| Negative control group | - | - | - |
| Salinomycin | 500 | 250 | 500 |
| Diclazuril | >500 | 500 | >500 |
| Xanthone | >500 | >500 | >500 |
| Mangosteen pericarp extract | 10 | 50 | 50 |

As shown in Table 11, in the xanthone treatment group, there was no direct killing effect of 100% against the protozoa capable of inducing coccidium up to 500ppm, and in case of diclazuril, against *Eimeria tenella* and *Eimeria maxima,* there was no direct killing effect of 100% against the protozoa up to 500ppm. In the mangosteen extract, at a significantly lower concentration than other groups, 100% of *Eimeria tenella, Eimeria acervulina* and *Eimeria maxima* could be killed, so it could be confirmed that the protozoal killing effect was significantly excellent.

[Table 12]

| Eimeria tenella protozoal death rate | | | |
|---|---|---|---|
| ppm | Mangosteen pericarp extract | Epicatechin | Alpha-mangostin |
| 15.6 | 100 | 0 | 93.3 |
| 7.8 | 93.3 | 0 | 50 |
| 3.9 | 40 | 0 | 23.3 |

As shown in Table 12, the mangosteen extract could kill 100% of the Eimeria tenella protozoan at the significantly lower concentration, 15.6ppm than the individual components comprised in the mangosteen, epicatechin and alpha-mangosteen, so it could be confirmed that the protozoal killing effect was significantly excellent. By LC-MS analysis (LC-MS analysis conditions are as follows: LC was waters acquity UPLC i-class, and for MS, waters xevo G2-XS(Q-TOF) product was used. For the used column information, waters acquity BEH C18 column was used.), the components and contents in the mangosteen extract were analyzed and this result was shown in Table 13. As shown in Table 13, the content of the alpha-mangostin in the mangosteen extract was at a level of about 12%, and considering this, the anticoccidial effect of the mangosteen extract itseft was superior to that of the alpha-mangostin.

[Table 13]

| Active ingredient in mangosteen extract (%) | |
|---|---|
| Alpha mangostin | 12.3% |
| Gamma mangostin | 1.06% |
| Quercetin | 0.008% |

(continued)

| Active ingredient in mangosteen extract (%) | |
|---|---|
| Epicatechin | 0.03% |

**Example 3. Example 3. Cell invasion and intracellular propagation inhibitory effect against *Eimeria* protozoa of mangosteen**

In the present example, using MDBK cell line which is a representative animal cell known to cause *Eimeria* infection and propagation, the inhibition ability of intracellular protozoan invasion and intracellular protozoal propagation of the mangosteen was investigated.

**Example 3-1. Cell invasion inhibitory effect of protozoan according to mangosteen treatment**

100,000 MDBK cells (purchased from ATCC) were aliquoted in a 24-well plate, and then incubated at a temperature of 37°C for 12 hours. The protozoan of *Eimeria acervulina* was obtained similar to the method of Example 2 above. 200,000 protozoa per one well were added to wells in which cells were aliquoted, and each material (mangosteen, xanthone and anticoccidial agents, salinomycin and diclazuril) was treated to the cells by concentration (1 or 10ppm), and cultured at a temperature of 41°C for 24 hours. A negative control group is a MDBK cell infected by protozoa, and a positive control group means a group in which salinomycin or diclazuril solution was incubated with the protozoan. Thereafter, in order to remove the protozoa that did not invade cells, the cells were washed twice using PBS solution. After removing the cells and protozoa inside the cells through pipetting, DNA was extracted from the cells, and PCR was performed using *E.acervulina ACE* gene-specific primers. The sequences of the used primers were described in Table 14 below.

[Table 14]

| Primer | | Nucleotide sequence (5'->3') | SEQ ID NO |
|---|---|---|---|
| *E.tenella ITS-1* | Forward | TGGAGGGGATTATGAGAGGA | SEQ ID NO: 1 |
| | Reverse | CAAGCAGCATGTAACGGAGA | SEQ ID NO: 2 |
| *E.acervulina ACE* | Forward | GCAGTCCGATGAAAGGTATTTG | SEQ ID NO: 3 |
| | Reverse | GAAGCGAAATGTTAGGCCATCT | SEQ ID NO: 4 |

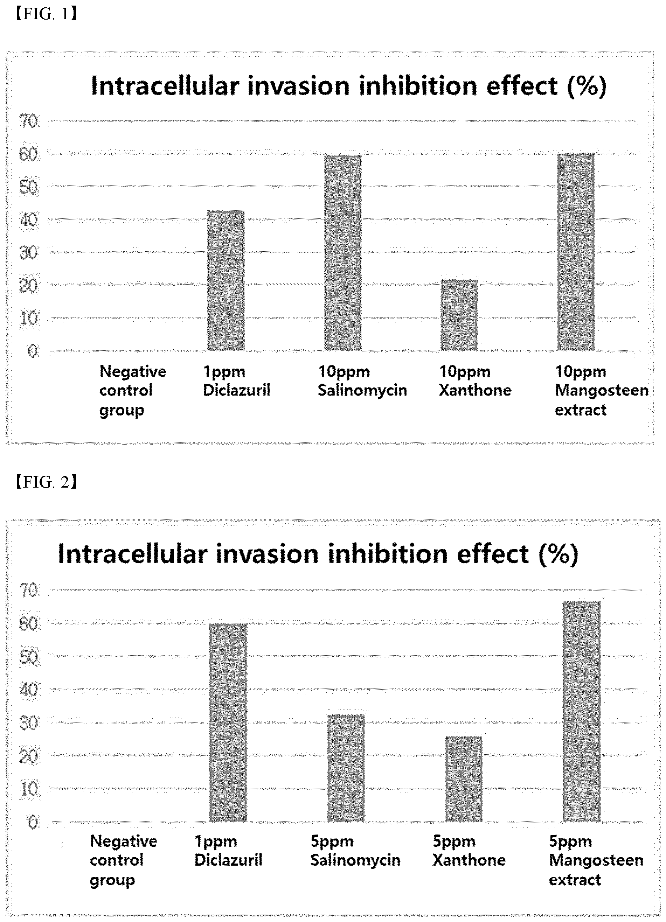

Ct values before/after washing for each material were compared and corrected with the ΔCt value of the negative control group to calculate the cell invasion inhibition rate (%) of the protozoa through treatment of each material, and the result was shown in FIG. 1. As shown in FIG. 1, it could be confirmed that the cell invasion inhibition efficacy against *Eimeria acervulina* in the mangosteen extract treatment group was excellent.

**Example 3-2. Intracellular propagation inhibition effect of protozoan according to mangosteen treatment**

100,000 MDBK cells (purchased from ATCC) were aliquoted in a 24-well plate, and then incubated at a temperature of 37°C for 12 hours. The protozoan of *Eimeria tenella* was obtained similar to the method of Example 2 above. 200,000 protozoa per one well were added to wells in which cells were aliquoted, and cultured at a temperature of 41°C for 24 hours, and then the cells were washed using PBS solution twice to remove the protozoa which were not attached to the cells. Each material (mangosteen, xanthone and anticoccidial agents, salinomycin and diclazuril) was treated to the cells by concentration, and further cultured at a temperature of 41°C for 24 hours. A negative control group is a MDBK cell infected by protozoa, and a positive control group means a group in which salinomycin and diclazuril solution was incubated with the protozoa. After removing the cells and protozoa propagated inside the cells through pipetting, DNA was extracted from the cells, and PCR was performed using *E. tenella ITS-1* (Internal transcribed spacer-1) gene-specific primers. The sequences of the used primers were described in Table 14 above.

The Ct values in the material treatment groups compared to the negative control group were compared to calculate the intracellular protozoan (sporozoite) propagation inhibition rate (%), and the result was shown in FIG. 2.

As shown in FIG. 2, the mangosteen extract treatment group showed a significantly excellent intracellular protozoal propagation inhibition effect of *E. tenella* than other groups.

**Example 4. Evaluation of cytotoxicity of mangosteen**

In the present example, using MTS analysis method (3-(4,5-dimethyl-2-yl)-5-(3-carboxymethoxyphenyl)-2-(4-sulfophenyl)-2H-tetrazolium, promega, USA), an effect on survival of cells of the mangosteen extract was investigated.

The cell lines utilized in cytotoxicity evaluation were (1) MDBK(Madin-Darby Bovine Kidney Epithelial Cells) and (2) RAW264.7(Mouse macrophage cell), and all of them were purchased from ATCC and used.

Each cell was aliquoted at a concentration of $1 \times 10^5$ cell/well in a 96-well cell culture plate, and cultured at 37°C for 24 hours, and then each material (mangosteen extract, xanthone, alpha-mangostin and epicatechin) was treated by concentration (3.1 ~ 500ppm/well) and cultured at a temperature of 37°C. After 14 hours, MTS solution was added to the cell culture solution, and incubated for 2 hours, and the absorbance was measured at a wavelength of 490nm by a microplate reader, and the cell survival rate (%) compared to the 5% DMSO treatment group, which was a negative control group, was calculated, and the cytotoxic minimum concentration where cells of 50% or more were survived was measured, and the result was shown in Table 15 below. In addition similar to the method of Example 2-1 above, the concentration of killing 100% of the *Eimeria tenella* protozoa of each material was measured, and the result was shown in Table 15.

As shown in Table 15, it could be confirmed that the cell safety was excellent as the mangosteen extract did not show cytotoxicity up to about 62.5ppm, and also, it could be confirmed that the mangosteen extract showed an excellent protozoal killing effect at a significantly lower concentration than the concentration at which cytotoxicity was shown.

[Table 15]

| Minimum concentration of cytotoxicity at which cells of 50% or more survive (ppm) | | | | |
|---|---|---|---|---|
| Cell | Alpha mangosteen | Xanthone | Epicatechin | Mangosteen extract |
| MDBK | ≥7.8 | ≥62.5 | ≥500 | ≥62.5 |
| RAW264.7 | ≥15.6 | ≥62.5 | ≥500 | ≥62.5 |
| Minimum concentrataion of killing protozoa 100% (ppm) | | | | |
| *Eimeria tenella* | ≥31.3 | ≥500 | ≥500 | ≥10 |

**Example 5. Evaluation of acid resistance of mangosteen**

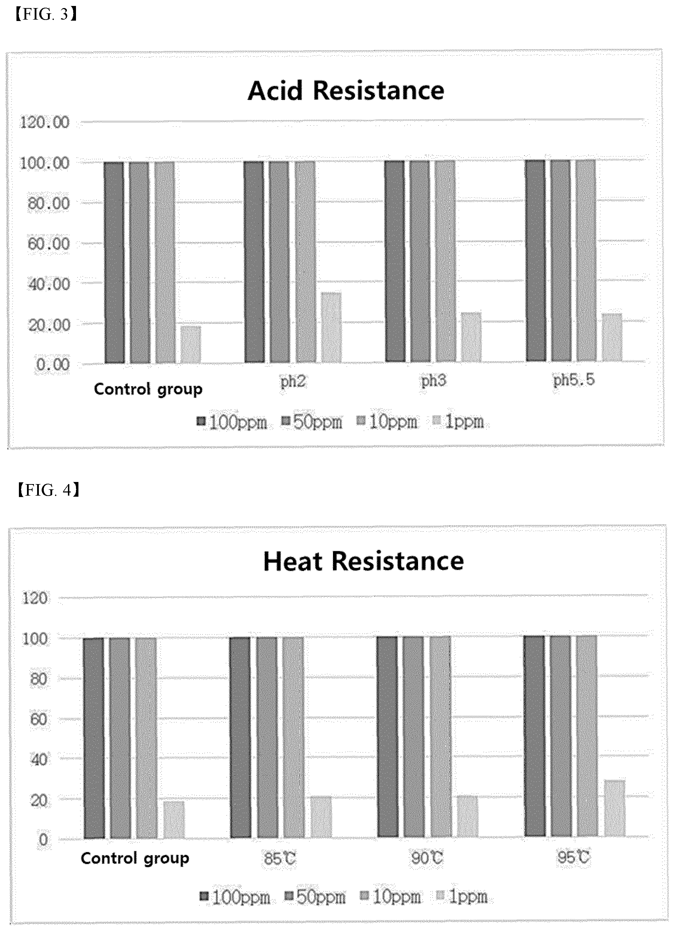

In the presen example, the acid resistance of the mangosteen itselt to be evaluated. Hydrochloric acid (HCl) solution was added to the mangosteen extract prepared in Example 1-2 above, to adjust to be pH 2, 3 and 5.5, and then it was left at a temperature of 40°C for 1 hour. Then, it was neutralized (pH 7.0) by adding sodium hydroxide (NaOH) solution, and 200,000 *Eimeria acervuilna* (*E. acervuilna*) protozoa (sporozoites) were exposed to the ginkgo leaf powder solution at various concentrations of 1 to 100ppm diluted using PBS solution, and they were reacted at a temperature of 41°C for 4 hours. Thereafter, the protozoal death rate (%) was measured by the similar method to Example 2 above, and this was shown in FIG. 3. As shown in FIG. 3, it could be confirmed that the mangosteen extract did not lose the killing efficacy against the protozoa inducing coccidiosis even under a strong acidic condition.

**Example 6. Evaluation of heat resistance of mangosteen**

In the present example, the heat resistance of the mangosteen was to be evaluated. The mangosteen extract prepared in Example 1-2 above was exposed at a temperature of 85 to 95°C for 10 minutes, and then heat was cooled and it was diluted with PBS solution at a concentration of 1, 10, 50, 100ppm. 200,000 *Eimeria acervuline* (*E. acervulina*) protozoa were reacted to the mangosteen extract at various concentrations of 1 to 100ppm at a temperature of 41°C for 4 hours, and the protozoal death rate (%) was measured by the similar method to Example 2 above, and this was shown in FIG. 4.

As shown in FIG. 4, it could be confirmed that the mangosteen extract did not lose the killing efficacy against the protozoa inducing coccidiosis even under an adverse condition of high temperature.

**Example 7. Evaluation of broiler growth effect due to mangosteen extract intake**

In the present example, an effect (improvement or degradation) on broiler growth of the mangosteen extract to be evaluated.

**Example 7-1. Experimental favility**

An efficacy evaluation test was performed in an animal experiment facility in Gyeongsangnam-do, South Korea. One-day-old female Ross broilers were individually weighed and randomly divided into groups to use in an experiment. Matters and conditions for experimental design were described in Table 16.

[Table 16]

| Category | Experiment variable |
|---|---|
| Breeding type | Cage |
| Broiler stocking age | 1-day-old |
| Total experiment period | 14 days |
| Gender | Female |
| Number of broilers per cage | 20 |
| Number of repetitions per treatment group | 2 repetitions |
| Number of treatment groups | 6 |
| Total number of broilers | 240 broilers |

A breeding farm was managed according to the Korean poultry breeding management guidelines. Cages and the breeding farm were cleaned and disinfected before starting the test. The temperature of 40 to 41°C and the humidity of 40 to 50% of the breeding farm were maintained, and it was continuously monitored.

**Example 7-2. Experimental design**

For the feed, Korea Feed A1-choi product was used, and each material (salinomycin (Cheil Bio Cheilsalino-60 product), xanthone (Sigma Aldrich X600 product), and the mangosteen extract prepared in Example 1-2 above) was added to the feed, respectively, at a concentration described in Table 17 below to be self-mixed. Antibiotics and supplements were not used in general feed and mixed feed, and no anticoccidial agent other than each material was added. Broilers were fed ad libitum throughout the experiment period. After putting 20 1-day-age broilers in cages randomly placed for each of the control group or test group and breeding, general feed was fed for 7 days, and then the mixed feed prepared above was ingested by dividing by control group or test group. The feed formulation administered to the control group (negative control group or positive control group) and test group was described in Table 17 below.

[Table 17]

| Group | Treatment group |
|---|---|
| Negative control group | General feed |
| Salinomycin treated group (positive control group) | Formulated feed comprising salinomycin 60ppm |
| Xanthone treated group 1 | Formulated feed comprising xanthone 250ppm |
| Xanthone treated group 2 | Formulated feed comprising xanthone 125ppm |
| Mangosteen extract treated group | Formulated feed comprising mangosteen extract 125ppm |

**Example 7-3. Calculation of daily weight gain of broilers**

The body weight before feeding and on the 7th day after feeding the feed of Table 16 above into individuals was measured, and the difference was divided by the number of days to calculate the daily weight gain (ADG, g/d), and the result was shown in Table 18. For comparison between 6 treatment groups, Duncan post-test multiple comparison method was used, and statistical significance was verified using a SAS statistical program, and the result was shown in Table 18.

As shown in Table 18, in the mangosteen extract treated group, compared to the negative control group, positive control group and xanthone treated group, the daily weight gain was significantly increased. On the other hand, in case of the xanthone treated group, the daily weight gain was rather reduced compare to the negative control group.

[Table 18]

| Treatment group | Initial body weig ht (g) | End body weight (g) | End-initial body weight (7days, g) | Daily weight gain (ADG) | Duncan p<0.05 |
|---|---|---|---|---|---|
| Negative control group | 193 | 512 | 319 | 45.57 | be |
| Salinomycin treated group (positive control group) | 198 | 526 | 328 | 46.86 | ab |
| Xanthone treated group 1 | 201 | 485 | 284 | 40.57 | d |
| Xanthone treated group 2 | 202 | 509 | 307 | 43.86 | bed |
| Mangosteen extract treated group | 208 | 539 | 331 | 47.29 | a |

**Claims**

1.  A feed composition for preventing or alleviating coccidioisis, comprising a mangosteen.

2.  The feed composition according to claim 1, wherein the mangosteen is mangosteen pericarp, mangosteen flesh, or a combination thereof.

3.  The feed composition according to claim 1, wherein the mangosteen is at least one selected from the group consisting of raw material, dried material, pulverized material and extract of the mangosteen.

4.  The feed composition according to claim 3, wherein the mangosteen extract is extracted by a solvent extraction method using an extraction solvent selected from the group consisting of water, linear or branched chain alcohols having 1 to 4 carbon atoms, propylene glycol, butylene glycol, glycerin, acetone, ethyl acetate, butyl acetate, chloroform, diethyl ether, dichloromethane, hexane and mixtures thereof.

5.  The feed composition according to claim 4, wherein the mangosteen extract is a mangosteen pericarp extract.

6.  The feed composition according to claim 1, wherein the coccidiosis is induced by an *Eimeria* sp. protozoan.

7.  The feed composition according to claim 1, wherein the preventing or alleviating coccidiosis is at least one selected from the group consisting of the following (1) to (4):

    (1) reduction of at least one selected from the group consisting of lesion score, fecal oocyst excretion amount and mortality;
    (2) inhibition of weight loss due to coccidiosis;
    (3) increase of an anticoccidial index (ACI); and
    (4) reduction of cell invasion of an *Eimeria* sp. protozoan, propagation of the protozoan in cells, or both of them.

8.  The feed composition according to claim 1, wherein the feed composition is a feed comprising the mangosteen at a concentration of 1%(w/w) or less based on the total weight.

9.  The feed composition according to any one of claims 1 to 7, wherein the feed composition is a feed additive.

10. A pharmaceutical composition for preventing or treating coccidiosis, comprising a mangosteen.

11. An antiprotozoal composition against an *Eimeria* sp. protozoan, comprising a mangosteen.

12. A method for preventing, alleviating or treating coccidiosis, comprising a step of administering a composition selected from the group consisting of the feed composition according to any one of claims 1 to 8; the pharmaceutical composition of claim 10; and the antiprotozoal composition according to claim 11 to an animal except for human.

【FIG. 1】

Intracellular invasion inhibition effect (%)

【FIG. 2】

Intracellular invasion inhibition effect (%)

【FIG. 3】

**Acid Resistance**

Control group    ph2    ph3    ph5.5

■100ppm  ■50ppm  ■10ppm  ■1ppm

【FIG. 4】

**Heat Resistance**

Control group    85℃    90℃    95℃

■100ppm  ■50ppm  ■10ppm  ■1ppm

## INTERNATIONAL SEARCH REPORT

| International application No. |
| --- |
| **PCT/KR2021/013035** |

**A.  CLASSIFICATION OF SUBJECT MATTER**

**A23K 20/10**(2016.01)i; **A23K 10/30**(2016.01)i; **A61K 36/38**(2006.01)i; **A61P 33/10**(2006.01)i; **A61P 33/04**(2006.01)i

According to International Patent Classification (IPC) or to both national classification and IPC

**B.  FIELDS SEARCHED**

Minimum documentation searched (classification system followed by classification symbols)

A23K 20/10(2016.01); A23K 20/121(2016.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 망고스틴(mangosteen), 콕시듐증(coccidiosis), 아이메리아(eimeria)

**C.  DOCUMENTS CONSIDERED TO BE RELEVANT**

| Category* | Citation of document, with indication, where appropriate, of the relevant passages | Relevant to claim No. |
| --- | --- | --- |
| A | KR 10-2018-0128190 A (DONGGUK UNIVERSITY INDUSTRY-ACADEMIC COOPERATION FOUNDATION) 03 December 2018 (2018-12-03)<br>See claims 1 and 4. | 1-12 |
| A | DHARMARATNE, H. R. W. et al. Antibacterial activity of xanthones from Garcinia mangostana (L.) and their structure-activity relationship studies. Natural Product Research. 2013, vol. 27, no. 10, pp. 938-941.<br>See entire document. | 1-12 |
| A | WIDOWATI, W. et al. Anti-inflammatory effect of mangosteen (Garcinia mangostana L.) peel extract and its compounds in LPS-induced RAW264. 7 cells. Natural Product Sciences. 2016, vol. 22, no. 3, pp. 147-153.<br>See entire document. | 1-12 |
| A | JANARDHANAN, S. et al. Antimicrobial effects of Garcinia mangostana on cariogenic microorganisms. Journal of clinical and diagnostic research: JCDR. 2017, vol. 11, no. 1, pp. ZC19-ZC22.<br>See entire document. | 1-12 |
| A | LEE, S.-H. et al. Immunomodulatory properties of dietary plum on coccidiosis. Comparative immunology, microbiology and infectious diseases. 2008, vol. 31, no. 5, pp. 389-402.<br>See entire document. | 1-12 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **28 December 2021** | **28 December 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

INTERNATIONAL SEARCH REPORT
Information on patent family members

International application No.

**PCT/KR2021/013035**

| Patent document cited in search report | Publication date (day/month/year) | Patent family member(s) | Publication date (day/month/year) |
|---|---|---|---|
| KR   10-2018-0128190   A | 03 December 2018 | KR       10-2160798   B1 | 29 September 2020 |

Form PCT/ISA/210 (patent family annex) (July 2019)

## REFERENCES CITED IN THE DESCRIPTION

**Patent documents cited in the description**

- KR 1020200125245

- US 20080160000 A

**Non-patent literature cited in the description**

- **WILLIAMS RB.** A compartmentalised model for the estimation of the cost of coccidiosis to the world's chicken production industry. *Int J Parasitol.,* 1999, vol. 29 (8), 1209-1229

- **JOYCE JOHNSON ; W.MALCOLM REID.** Anticoccidial drugs: Lesion scoring techniques in battery and floor-pen experiments with chickens. *Experimental parasitology,* 1970